# EUROPEAN PATENT SPECIFICATION

(11) **EP 0 310 430 B1**
(45) Date of publication and mention of the grant of the patent: **04.05.1994**
(21) Application number: 88309122.5
(22) Date of filing: 30.09.1988
(51) Int. Cl.: G01N 33/78, G01N 33/58

(54) **Homogeneous T-3 or T-4 uptake assay method and kit**
Homogenes Verfahren zur Bestimmung der T-3- oder T-4-Aufnahme und Zusammensetzung dafür
Procédé homogène pour déterminer l'absorption de T-3 ou T-4 et composition pour cela

(30) Priority: 02.10.1987 US 104342
(43) Date of publication of application: 05.04.1989
(73) Proprietor: MICROGENICS CORPORATION, Concord California 94520 (US)
(72) Inventor: Picone, Teresa K., Vallejo, CA 94590 (US); Friedman, Stephan B., Chapel Hill, NC 27516 (US)
(74) Representative: Harrison, David Christopher

(56) References cited:
- EP-A- 0 062 277
- US-A- 4 171 244
- US-A- 4 341 865
- US-A- 4 708 929
- CLINICAL CHEMISTRY, Theory, Analysis & Correlation, 1984, Chen & Sperling; C.V. MOSBY et al., pp. 1170-1173#

## Description

The present invention relates to diagnostic assays, and in particular to an assay for thyroid uptake.

The active thyroid hormones are designated T-4 and T-3. In the blood, T-4 and T-3 are almost entirely bound to plasma proteins. Thyroxine binding globulin (TBG) is normally the major determinant of overall binding intensity of T-4. The interaction between T-4 and its binding proteins is in reversible binding equilibrium. Only a small portion, usually about 0.03 percent, of T-4 is free in blood. However, T-3 is not as readily bound, and as a consequence, the normal proportion of free T-3 is eight to ten times greater than that of T-4. Only free (unbound) hormone is available to tissues. Therefore, the metabolic state of the patient correlates more closely with the concentration of free hormone, rather than the total concentration of hormone, in plasma.

There are two general types of disturbances of the thyroid hormone-plasma protein interaction. In the first type of disturbance, an increased amount of TBG or other serum binding proteins or the appearance of abnormal serum binding proteins results in a lowered free hormone level. Total hormone concentration in serum may then increase until the concentration of free hormone is restored to normal. In the second type of disturbance, the concentration of thyroxine-binding globulin (TBG) is normal, but the amount of thyroid hormone is abnormal. In this type of disorder, the proportion of free hormone changes in the same direction as the change in hormone supply.

When the amount of free T-4 is too low, patients can be treated with synthetic T-4 (levothyroxine). In that case it is important to determine how much administered synthetic T-4 will be bound by TBG, rather than the total thyroxine concentration after administration of T-4, since only unbound hormone is effective in treating the patient.

A number of assays related to hormone concentration and binding in blood have been developed. Free T-4 concentration can be measured by equilibrium dialysis of serum enriched with a small amount of labeled T-4. The percent of T-4 that is dialyzable, and therefore free, is determined. From that number, the percentage that is bound can be calculated. However, the dialysis technique is cumbersome and has not generally been useful for clinical purposes. An in vitro uptake test in which serum is incubated with labeled T-4 or T-3 and then with an insoluble particulate matter such as resin or charcoal that non-specifically binds free hormone has also been developed. The percentage of labeled hormone associated with the particulate matter varies inversely with the concentration of unoccupied sites among serum proteins and their affinity for the labeled hormone.

Methods which would more accurately determine uptake values of TBG would assist the clinician in differentiating low T-4 state of a non-thyroidal illness from overt thyroxine deficiency of hypothyroidism. Such methods would also allow the clinician to more accurately determine the amount of synthetic T-4 to administer to alleviate symptoms produced by hypothyroid disease.

Kaptein et al., J. of Clin. Endrocrinol. and Metab. (1981) 52:1073 provides a comparison of eight methods of making free thyroxine estimates. The article describes the ability of the assays to distinguish low T-4 levels caused by hypothyroid and hypothalamic-pituitary diseases from those caused by non-thyroidal illnesses. The evaluated assay methods were equilibrium dialysis, enzyme immunoassay (Abbott), antibody-coated tube (Clinical Assays), antibody-coated microfine silica (Corning Immunophase), microencapsulated antibody (Damon) and free T-4 uptake using the T-3 uptake ratio or the thyroxine binding globulin method.

WO-A-86/02666 (= US-A-4708929) describes an enzyme acceptor/enzyme-donor technique for enzyme complementation assays. TBG is mentioned as an example of a type of binding protein used in the method, not as analyte.

The present invention provides an improved method of measuring thyroxine uptake in which thyroxine binding globulin (TBG) activity is measured directly. A method of measuring thyroxine uptake by TBG in a sample comprises combining the sample in an aqueous solution with an enzyme donor (ED) conjugated to T-3 or T-4 or an analogue thereof that binds with available thyroxine binding globulin sites. Attachment of T-4 to ED is controlled so that the affinity of TBG for T-4 is not altered significantly. The sample is further combined with an enzyme acceptor (EA) characterized by providing a modulated enzyme activity in relation to the amount of TBG activity. The amount of enzyme activity in comparison to a control solution having a known amount of available TBG binding sites is determined.

The instant method is faster, more precise, and simpler to perform in addition to being more accurate and economical than indirect measurement methods in which binding is measured using antibodies.

An improved method is provided of measuring thyroxine uptake in which thyroxine binding globulin (TBG) activity is measured directly. The present method provides reliable information as to whether low levels of free T-3 and/or T-4 in the serum are due to hypothyroidism or to non-thyroidal illness.

The method comprises combining a sample in an aqueous solution with an enzyme donor conjugated to T-3 or T-4 or analogue thereof that binds with available thyroxine binding globulin sites. The enzyme acceptor, the enzyme donor and enzyme substrate are combined with the sample to form an assay medium. The sample may be pre-incubated with either the enzyme donor conjugate or the enzyme acceptor. Alternatively, all components of the assay medium may be combined so long as the enzyme donor and the enzyme acceptor are not mixed in the absence of sample. The enzyme acceptor is characterized, in part, by providing a modulated enzyme activity in relation to the amount of TBG activity when bound to the conjugate. That is, the amount of enzyme activity decreases as the TBG activity of the sample increases. The amount of enzyme activity is compared to a control solution having a known amount of thyroxine uptake by TBG.

The assay method can be used with any sample containing thyroxine binding globulin. Usually the sample will be patient serum or plasma. Other than removal of particulates, no other pretreatment of the sample will usually be performed for purposes of the instant assay method.

The enzyme donor and enzyme acceptor are partial sequences of β-galactosidase, the enzyme donor being smaller. The enzyme donor and the enzyme acceptor can be mutated. The β-galactosidase enzyme donor fragment has substantially the same amino acid sequence as the N-terminal portion of the β-galactosidase monomer, the enzyme acceptor has substantially the same or the same sequence as the C-terminus, being more than 50% of the β-galactosidase monomer molecule. β-Galactosidase activity results upon complementation of the enzyme donor conjugate with the β-galactosidase enzyme acceptor monomer fragment.

The enzyme acceptor is characterized by providing in conjunction with the enzyme donor conjugate a complex whose enzyme activity varies in relation to the amount of thyroxine binding globulin activity under the conditions of the assay. That is, when the enzyme donor conjugate is bound to TBG, the enzyme activity during the time of measurement is substantially reduced in comparison to the enzyme activity when the enzyme donor conjugate is unbound.

The enzyme donor fragment is conjugated to T-3, T-4 or an analogue thereof that binds to thyroxine binding globulin binding sites. The analogue is usually T-4 or related to T-4. T-3 may also be used; however, TBG has a much lower affinity for T-3, depending on the ED used, leading to less accurate measurements. For the most part, the analogue will have a 4-(3′,5′-diiodo-4′-hydroxyphen-1′-oxy)-3,5-diiodophenyl group.

β-galactosidase enzyme donors and acceptors are described in WO-A-86/02666 (= US-A-4 708 929)
The aqueous solution and assay conditions provide for complementation between enzyme donor and enzyme acceptor. In general, physiological buffers such as sodium phosphate buffer are useful. A preferred buffer comprises 10 to 30 mM NaPO₄, 5 to 10 mM EGTA, and 10 to 20 mM NaN₃ having a pH of between 6 and 8. The temperature will usually be at least at 25°C, preferably elevated, but below 60°, usually below 70°C. Enzyme assays are generally conducted at room temperature (25° C) to less than 40° C, usually about 37°C. The assays are performed at atmospheric pressure.

The concentration of enzyme donor conjugates in the assay medium will usually be in the range of about 1 to 60 nM, more usually 5 to 25 nM, while the enzyme acceptor concentration will be 40 to 200 units/ml, usually 80 to 120 units/ml. The molar ratios of enzyme donor conjugate to enzyme acceptor will usually be 1:30 to 1:80 usually 1:50 to 1:60. The usual range of interest for quantitation of TBG binding sites will be 20 to 50% uptake.

The amount of enzyme activity in the assay medium will be determined in accordance with standard techniques for measuring β-galactosidase activity. An enzyme substrate is employed that when cleaved by the enzyme results in a change in the amount of light absorbance or emission of the assay medium. That is, cleavage of the substrate results in the appearance or disappearance of a colored or a fluorescent product. A preferred enzyme substrate is ortho-nitrophenyl-β-galactoside (ONPG) which is enzymatically cleaved to form ortho-nitrophenol (ONP). ONP absorbance values are measured at 420 nm. Other comparable enzyme substrates are also commercially available.

Determining the amount of TBG activity in comparison to a sample having a known amount of TBG activity can be performed in a number of ways. Conveniently, the rate of change of absorbance in a known sample can be compared to the rate of change in the unknown. One or more unknown samples and a control sample (standard), usually two or three standards, are assayed. The sample(s) and the controls are processed in parallel in the following way. An initial reading at about 3 to about 4 minutes after mixing the sample is performed. The samples are incubated for an additional 1 to 2 minutes. The second absorbance values are read. The change in absorbance or emission values of the standards can be used to prepare a standard curve. The unknown TBG binding site value is determined in relation to the standard curve. In this way, the percentage of T-4 uptake in a sample can be determined from the curve.

A kit containing reagents facilitating the present invention is also contemplated. The kit comprises in at least one container, usually separate containers, a β-galactosidase enzyme donor conjugate as described above, and an enzyme acceptor. When packaged in separate containers, the enzyme donor or enzyme acceptor may additionally contain enzyme substrate. The enzyme donor conjugate and enzyme acceptor and substrate may be present in lyophilized form. The kit will usually contain a buffer suitable for reconstituting the reagents, which may be packaged with the enzyme components or separately. The kit may also contain one or more control samples comprising serum having a known amount of thyroxine uptake by thyroxine binding globulin. The kit can be particularly designed for use with autoanalyzers.

The following examples are offered by way of illustration for purposes of clarity and not by way of limitation.

### EXPERIMENTAL

Buffered solutions were prepared of 11 nM enzyme donor conjugate (ED4-T₄, see Application Serial No. 721,267, supra) in ED buffer (20 mM NaPO₄; 10 mM EGTA (ethylene-bis-oxyethylenenitrilo tetraacetic acid); 20 mM NaN₃; .05% Tween 20; 6.25 mg/ml ONPG; 0.9% N-lauroylsarcosine, Na salt; pH 7.0) and 22 units of enzyme acceptor (EA-22) in EA buffer (20 mM NaPO₄; 10 mM EGTA: 20 mM NaN₃; 5 mM Sucrose; 2 mM Mg(OAC)₂ 4 H₂O; .2% β-cyclodextrin; 3.6% Glycerol; pH 7.0). Both solutions were stored on ice until used. Additionally, control serum samples were used as calibrators. The value of the calibrators was 20, 35 and 50 percent T-uptake. Two patient samples having an unknown TBG activity were also assayed.

14 µl of each known serum standard was pipetted into a labeled well of a 96 well microtiter plate. 14 µl of each unknown serum was also added to a well of a 96 well microtiter plate. 14 µl of distilled water was added to one well to serve as a blank. 36 µl of distilled water was added to each microtiter well. 160 µl of enzyme acceptor solution was added to each well except the blank which received 160 µl of distilled water. The solutions were mixed by either tapping the plate or rotating the plate. Thereafter, 40 µl of enzyme donor solution was placed in each well. The solutions were mixed by tapping or rotating the plate. The plate was placed in a 37°C incubator for four minutes. The microtiter plate was removed from the incubator and absorbance values at 420 nm were read for each sample using a microtiter plate reader with the appropriate filter. Immediately after reading the plate, it was returned to the incubator. A second absorbance reading for each well was taken one minute after the first reading, five minutes total time and the difference between the values determined. A standard curve for the known standards using known uptake values as the X-axis and the one-minute rate as the Y-axis was constructed. The rate of the unknown sample was read off of the curve to determine the percentage of T-uptake.

The above results demonstrate an easy, accurate and rapid direct determination of available binding sites of TBG. The method does not require indirect determinations involving excess thyroxine to equilibriate the specimen and determine total binding site in addition to determining total thyroxine nor the use of radioisotopes. The TBG is able to bind to the enzyme donor conjugate so as to permit a direct measurement of available TBG sites.

## Claims

1. A method for directly measuring available thyroxine binding sites in a sample, comprising:
(a) combining in an assay medium (1) a β-galactosidase substrate, (2) said sample, (3) a β-galactosidase acceptor fragment having substantially the same amino acid sequence as a C-terminus region of β-galactosidase, and (4) a β-galactosidase enzyme donor conjugate comprising a β-galactosidase enzyme donor fragment, wherein said donor fragment has substantially the same amino acid sequence as an N-terminus region of β-galactosidase complementary to said C-terminus region, linked to the active thyroid hormone T-3 or T-4 or analogue thereof that competes with thyroxine for free thyroxine binding sites, with the proviso that said β-galactosidase donor conjugate and said β-galactosidase acceptor fragment are not both present in the absence of said sample, and wherein said donor and acceptor fragments form an active β-galactosidase enzyme upon complexation, said complexation being modulated if said enzyme donor conjugate binds to a protein having a thyroxine binding site; and
(b) determining the amount of β-galactosidase activity in said assay medium as compared to an assay medium containing a known amount of available thyroxine binding sites.

2. A method according to claim 1, wherein said enzyme is β-galactosidase and said enzyme donor fragment is the smaller fragment and comprises substantially the same sequence as the N-terminal sequence of β-galactosidase.

3. A method according to claim 2, wherein said sample is serum or plasma.

4. A method according to claim 1 wherein the β-galactosidase donor fragment is the smaller of the fragments and comprises substantially the same sequence as the N-terminus of β-galactosidase and the β-galactosidase acceptor fragment is the larger of the fragments, said method comprising:
(a) mixing in an aqueous solution to form an assay medium:
(i) said sample;
(ii) said acceptor fragment;
(iii) a donor conjugate comprising said donor fragment linked to an active thyroid hormone T-3 or T-4 or analogue thereof which is competing with thyroxine for said sites;
(iv) the β-galactosidase substrate, which undergoes a β-galactosidase catalysed reaction to produce a light absorbing or emitting product;
(b) incubating said assay medium for a sufficient time for complexation to occur;
(c) measuring the light absorption or emission at two different times and determining the difference in order to carry out the said comparison.

5. A method according to claim 4, wherein said two different times are spaced at least one minute apart.

6. A method according to claim 4, wherein said substrate is O-nitrophenyl-β-galactoside.

7. A method according to claim 4, wherein said competing active thyroid hormone or analogue comprises a 4-(3',5'-diiodo-4'-hydroxyphen-1'-oxy)-3,5-diiodophenyl group.

8. A kit for carrying out the method of claim 1 comprising (1) the said donor conjugate, (2) the said enzyme acceptor, (3) a control containing a known amount of thyroxine binding sites and (4) the said substrate.

9. A kit according to claim 8, wherein said (1) and (2) are lyophilized.

## Patentansprüche

1. Verfahren zum direkten Messen verfügbarer Thyroxinbindungsstellen in einer Probe, umfassend:
(a) das Kombinieren in einem Assaymedium (1) eines β-Galaktosidasesubstrats, (2) der genannten Probe, (3) eines β-Galaktosidaseakzeptorfragments, das im wesentlichen die gleiche Aminosäuresequenz wie eine C-Terminusregion von β-Galaktosidase aufweist, und (4) eines β-Galaktosidaseenzymdonorkonjugats, das ein β-Galaktosidaseenzymdonorfragment umfaßt, worin das genannte Donorfragment im wesentlichen die gleiche Aminosäuresequenz wie eine N-Terminusregion von β-Galaktosidase komplementär zur genannten C-Terminusregion an das aktive Thyroidhormon T-3 oder T-4 oder ein Analog davon gebunden aufweist, das mit Thyroxin um freie Thyroxinbindungsstellen konkurriert, mit der Maßgabe, daß das genannte β-Galaktosidasedonorkonjugat und das genannte β-Galaktosidaseakzeptorfragment in Abwesenheit der genannten Probe nicht beide vorhanden sind, und worin die genannten Donor- und Akzeptorfragmente nach Komplexbildung ein aktives β-Galaktosidaseenzym bilden, wobei die genannte Komplexbildung moduliert wird, wenn das genannte Enzymdonorkonjugat sich an ein Protein mit einer Thyroxinbindungsstelle bindet; und
(b) das Bestimmen der Menge an β-Galaktosidasewirksubstanz im genannten Assaymedium im Vergleich mit einem Assaymedium, das ein bekannte Menge verfügbarer Thyroxinbindunggstellen enthält.

2. Verfahren nach Anspruch 1, worin das genannte Enzym β-Galaktosidase ist und das genannte Enzymdonorfragment das kleinere Fragment ist und im wesentlichen die gleiche Sequenz wie die N-terminale Sequenz von β-Galatosidase aufweist.

3. Verfahren nach Anspruch 2, worin die genannte Probe Serum oder Plasma ist.

4. Verfahen nach Anspruch 1, worin das β-Galaktosidasedonorfragment das kleinere der Fragmente ist und im wesentlichen die gleiche Sequenz wie der N-Terminus von β-Galaktosidase aufweist und das β-Galaktosidaseakzeptorfragment das größere der Fragmente ist, wobei das genannte Verfahren umfaßt:
(a) das Mischen in einer wässerigen Lösung, um ein Assaymedium zu bilden, von:
(i) der genannten Probe;
(ii) des genannten Akzeptorfragments;
(iii) eines Donorkonjugats, welches das genannte Donorfragment an ein aktives Thyroidhormon T-3 oder T-4 oder ein Analog davon gebunden aufweist, das mit Thyroxin um die genannten Stellen konkurriert;
(iv) des β-Galaktosidasesubstrats, das eine β-Galaktosidase-katalysierte Umsetzung durchmacht, um ein lichtabsorbierendes oder -aussendendes Produkt zu erzeugen;
(b) das Inkubieren des genannten Assaymediums für ausreichende Zeit, damit Komplexbildung auftritt;
(c) das Messen der Lichtabsorption aus -aussendung zu zwei verschiedenen Zeitpunkten und das Bestimmen der Differenz, um den genannten Vergleich durchzuführen.

5. Verfahren nach Anspruch 4, worin die genannten beiden unterschiedlichen Zeitpunkte zumindest einen Abstand von einer Minute aufweisen.

6. Verfahren nach Anspruch 4, worin das genannte Substrat O-Nitrophenyl-β-galaktosid ist.

7. Verfahren nach Anspruch 4, worin das genannte konkurrierende aktive Thyroidhormon oder Analog eine 4-(3',5'-Dijod-4'-hydroxyphen-1'-oxy)-3,5-dijodphenylgruppe umfaßt.

8. Set zur Durchführung des Verfahrens nach Anspruch 1, das (1) das genannte Donorkonjugat, (2) den genannten Enzymakzeptor, (3) eine Kontrolle, die eine bekannte Menge an Thyroxinbindungsstellen enthält, und (4) das genannte Substrat umfaßt.

9. Set nach Anspruch 8, worin die genannten (1) und (2) lyophilisiert sind.

## Revendications

1. Procédé pour mesurer directement des sites disponibles de liaison de thyroxine dans un échantillon, comprenant les étapes de :
(a) combiner dans un milieu de test (1) un substrat de β-galactosidase, (2) ledit échantillon, (3) un fragment accepteur de β-galactosidase ayant substantiellement la même séquence acide aminé qu'une région C-terminus de β-galactosidase, et (4) un conjugué donneur d'enzyme de β-galactosidase comprenant un fragment de donneur d'enzyme de β-galactosidase, dans lequel ledit fragment donneur a substantiellement la même séquence acide aminé qu'une région N-terminus de la β-galactosidase complémentaire à ladite région C-terminus, lié à l'hormone thyroïdienne active T-3 ou T-4 ou un analogue de celle-ci qui est en compétition avec la thyroxine pour les sites de liaison de thyroxine, à la condition que ledit conjugué donneur de β-galactosidase et ledit fragment accepteur de β-galactosidase ne soient pas ensemble présents en l'absence dudit échantillon, et dans lequel lesdits fragments donneurs et accepteurs forment un enzyme actif de β-galactosidase lors de la complexation, ladite complexation étant modulée si ledit conjugué donneur d'enzyme se lie à une protéine ayant un site de liaison thyroxine; et
(b) déterminer la quantité d'activité de la β-galactosidase dans ledit milieu test comparée à un milieu test contenant une quantité connue de sites disponibles de liaison thyroxine.

2. Procédé selon la revendication 1, dans lequel ledit enzyme est du β-galactosidase et ledit fragment donneur d'enzyme est le plus petit fragment et comprend substantiellement la même séquence que la séquence N-terminale de la β-galactosidase.

3. Procédé selon la revendication 2, dans lequel ledit échantillon est du sérum ou du plasma.

4. Procédé selon la revendication 1, dans lequel le fragment donneur de β-galactosidase est le plus petit des fragments et comprend essentiellement la même séquence que le N-terminus de la β-galactosidase et le fragment accepteur de β-galactosidase est le plus grand des fragments, ledit procédé comprenant les étapes de :
(a) mélanger dans une solution aqueuse pour former un milieu test :
(i) ledit échantillon;
(ii) ledit fragment accepteur;
(iii) un conjugué donneur comprenant ledit fragment donneur lié à une hormone T-3 ou T-4 active de thyroïde ou un analogue de celle-ci, qui est en compétition avec la thyroxine pour lesdits sites;
(iv) le substrat de β-galactosidase, qui subit une réaction catalysée par la β-galactosidase pour produire un produit légèrement absorbant ou émetteur;
(b) incuber ledit milieu de test pendant un temps suffisant pour que la complexation se produise;
(c) mesurer l'absorption ou l'émission légère à deux temps différents et déterminer la différence de façon à mettre en oeuvre ladite comparaison.

5. Procédé selon la revendication 4, dans lequel lesdits deux temps différents sont espacés d'au moins une minute.

6. Procédé selon la revendication 4, dans lequel ledit substrat est du O-nitrophényl-β-galactoside.

7. Procédé selon la revendication 4, dans lequel ladite hormone active de thyroïde en compétition ou son analogue comprend un groupe 4-(3',5'-diiodo-4'-hydroxyphen-1'-oxy)-3,5-diiodophényle.

8. Nécessaire pour mettre en oeuvre le procédé de la revendication 1, comprenant (1) ledit conjugué donneur, (2) ledit accepteur d'enzyme, (3) une référence contenant une quantité connue de sites de liaison thyroxine et (4) ledit substrat.

9. Nécessaire selon la revendication 8 , dans lequel lesdits (1) et (2) sont lyophilisés.
